# EUROPEAN PATENT SPECIFICATION

(11) **EP 1 704 891 B1**
(45) Date of publication and mention of the grant of the patent: **22.06.2011**
(21) Application number: 06251505.1
(22) Date of filing: 21.03.2006
(51) Int. Cl.: A61M 39/02

(54) **Subcutaneous injection port with stabilizing elements**
Subkutane Injektionsöffnung mit Stabilisierungselementen
Orifice d'injection sous-cutanée avec éléments de stabilisation

(30) Priority: 22.03.2005 US 86195
(43) Date of publication of application: 27.09.2006
(73) Proprietor: ETHICON ENDO-SURGERY, INC., Cincinnati, Ohio 45242 (US)
(72) Inventor: Schulze, Dale R., Lebanon Ohio 45036 (US); Chen, How-Lun, Cincinnati Ohio 45242 (US)
(74) Representative: Tunstall, Christopher Stephen

(56) References cited:
- EP-A- 1 488 824
- WO-A-01/08597
- US-A- 5 207 644
- US-A- 5 688 247

## Description

**Field of the Invention**

This invention relates generally to the field of medicine, and more specifically to medical devices that are surgically implanted in a patient, and is particularly relevant to implantable injection or infusion ports such as used for chemotherapy and adjustable gastric band procedures.

**Background of the Invention**

Surgeons routinely implant subcutaneous injection ports in patients requiring periodic fluid injections such as for chemotherapy and gastric band adjustments. The injection port connects to a flexible tube catheter to transport the fluid to the affected area (subclavian vein, etc.) or the gastric band. Current injection ports comprise a rigid metal or plastic housing, which is about 25mm in diameter and 15mm tall. A thick, silicone septum captured within the rigid housing covers an inner chamber that fluidly communicates with the catheter. The surgeon uses a hypodermic needle to inject fluid into the chamber through the silicone septum.

Such injection ports are commonly use in conjunction with adjustable gastric bands to treat morbid obesity. Examples of an adjustable gastric band can be found in U.S. Patents 4,592,339 issued to Kuzmak; RE 36176 issued to Kuzmak; 5,226,429 issued to Kuzmak; 6,102,922 issued to Jacobson and 5,601,604 issued to Vincent. In accordance with current practice, a gastric band is operatively placed to encircle the stomach. This divides the stomach into two parts with a stoma in-between. An upper portion, or a pouch, which is relatively small, and a lower portion which is relatively large. The small partitioned portion of the stomach effectively becomes the patient's new stomach, requiring very little food to make the patient feel full.

Once positioned around the stomach, the ends of the gastric band are fastened to one another and the band is held securely in place by folding a portion of the gastric wall over the band and closing the folded tissue with sutures placed therethrough thereby preventing the band from slipping and the encircled stoma from expanding. Gastric bands typically include a flexible substantially non-extensible portion having an expandable, inflatable portion attached thereto. The inflatable portion is in fluid communication with such an injection site, or port. Injection or removal of an inflation fluid into or from the interior of the inflatable portion is used to adjust the size of the stoma either during or following implantation. By enlarging the stoma, the patient can eat more food without feeling as full, but will not lose weight as fast. By reducing the size of the stoma, the opposite happens. Physicians regularly adjust the size of stoma to adjust the rate of weight loss.

Most commercially available injection ports have holes spaced around the perimeter of the housing for suturing the port to the tissue. Attaching the port to tissue helps to prevent the port from flipping over and/or migrating in the body. When implanting the injection port for a gastric band, the surgeon typically fastens the injection port with four sutures to the fascia covering the abdominal musculature and beneath the fat layer, which may be several centimeters thick for obese patients. Since for most commercially available ports the septum is accessible from only one side of the injection port, flipping over may require interventional surgery to right the port for subsequent injections.

Currently many surgeons implant the gastric band and catheter using a laparoscopic procedure to minimize patient pain, cost, and recovery time. However, once the surgeon has implanted the gastric band and catheter, the surgeon may externalize the proximal end of the catheter through a peritoneal incision, fluidly connect the catheter to the injection port, and then use an open procedure to attach the injection port to the fascia over the abdominal musculature. Placement of the band around the stomach is a difficult and important part of the surgical procedure. Implantation of the injection port is no less critical to the overall success of the gastric band, but many surgeons regard this part of the procedure as routine and are anxious to complete it. In addition, suturing the injection port to tissue requires a large enough surgical incision for accessing the suturing site with dissecting instruments and needle graspers. The associated wound and tissue trauma may result in significant post-operative pain and recovery time for the patient. What is needed, therefore, is a subcutaneously implantable injection port that does not require suture attachment to tissue to prevent migration of the port and/or flipping over. It is important that such an injection port be positionable into soft tissue with minimal trauma to surrounding tissue. The port should allow quick healing of the surrounding wound and be comfortable and cosmetically acceptable to the patient.

EP 1 488 824 describes an implantable surgical injection port comprising a housing having a body, a closed distal end, an open proximal end, and a fluid reservoir between them, a needle penetrable septum retained in the open proximal end of the housing, and at least one stabilizing element mounted to the housing for stabilizing the port within tissue, the stabilizing element comprising a member having an un-deployed position and a deployed position. The stability elements are in the form of flexible fasteners that lie in an axial plane. The flexible fasteners are designed to penetrate tissues and act as anchors for the injection port described.

### Summary of the Invention

The present invention is an implantable surgical injection port including a housing having a body, a closed distal end, an open proximal end and a fluid reservoir therebetween. The housing includes a needle penetrable septum attached to the housing about the opening. The injection port further includes at least one stabilizing element mounted to the housing for stabilizing the port within tissue. The stabilizing element is a member having an undeployed position and a deployed position, wherein the element extends radially from the body in the undeployed position and, when deployed, lies in a plane that is substantially parallel to the septum.

### Brief Description of the Figures

We present the specific, novel features of this invention in the appended claims. The reader may best understand, however, the organization and methods of operation of this invention by referring to the detailed description and the following drawings:
FIG. 1 is a side view of an injection port 2 of the prior art;
FIG. 2 is a top view of injection port 2 of the prior art;
FIG. 3 is a perspective view of injection port 2, a connector 16, a ferrule 18, and a catheter 20, in general alignment for assembly and implantation through a bodily incision 24;
FIG. 4 is a perspective view of injection port 2 assembled to catheter 20 and attached to a tissue layer 26;
FIG. 5 is a side view of a first embodiment of an injection port 100 with radially extendable, stabilizing elements 102, shown in a deployed position;
FIG. 6 is a top view of injection port 100 in the deployed position;
FIG. 7 is a side view of injection port 100, shown in an undeployed position;
FIG. 8 is a top view of injection port 100, shown in the undeployed position;
FIG. 9 is a perspective, exploded view of the components of injection port 100;
FIG. 10 is a side view of a second embodiment of an injection port 200, shown in a deployed position;
FIG. 11 is a top view of injection port 200 in the deployed position;
FIG. 12 is a side view of injection port 200, shown in an undeployed position;
FIG. 13 is a top view of injection port 200 in the undeployed position;
FIG. 14 is a perspective, exploded view of injection port 200;
FIG. 15 is a side view of a third embodiment of an injection port 300, shown in a deployed position;
FIG. 16 is a top view of injection port 300 in the deployed position;
FIG. 17 is a top view of injection port 300 in an undeployed position;
FIG. 18 is a side view of injection port 300 in the undeployed position;
FIG. 19 is a perspective, exploded view of injection port 300;
FIG. 20 is a top view of a fourth embodiment of an injection port 400; shown in a deployed position;
FIG. 21 is a side view of injection port 400 in the deployed position;
FIG. 22 is a top view of a fifth embodiment of an injection port 500;
FIG. 23 is a side view of injection port 500;
FIG. 24 is a top view of a sixth embodiment of an injection port 600;
FIG. 25 is a side view of injection port 600;
FIG. 26 is a top view of a seventh embodiment of an injection port 700;
FIG. 27 is a side view of injection port 700;
FIG. 28 is a side view of an eighth embodiment of an injection port 800; and
FIG. 29 is a top view of injection port 800.

**Detailed Description of the Invention**

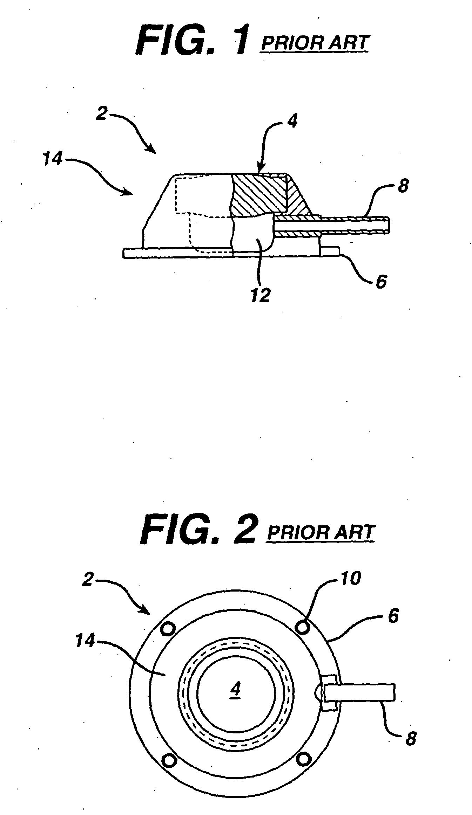

Referring now to the drawings, FIGS. 1 and 2 show an injection port 2 of the prior art. Injection port 2 generally may have a truncated, conical configuration, and comprises a housing 14, a septum 4, and a catheter support 8. Injection port 2 further comprises a body 7 having a bottom surface, also called a distal closed end 13, and an open proximal end 5, which retains septum 4. Housing 14 is typically made of a biocompatible, corrosion resistant metal. Septum 4 may be made of an elastomeric material such as silicone rubber, which is easily penetrable by a hypodermic needle. Housing 14 and septum 4 define a fluid reservoir 12 in injection port 2 for receiving and containing a fluid. Catheter support 8 extends through housing 14 to provide fluidic communication between fluid reservoir 20 and the exterior of injection port 2. A flange 6 extends from housing 14 and contains a plurality of holes 10 for suturing injection port 2 to the tissue of a patient.

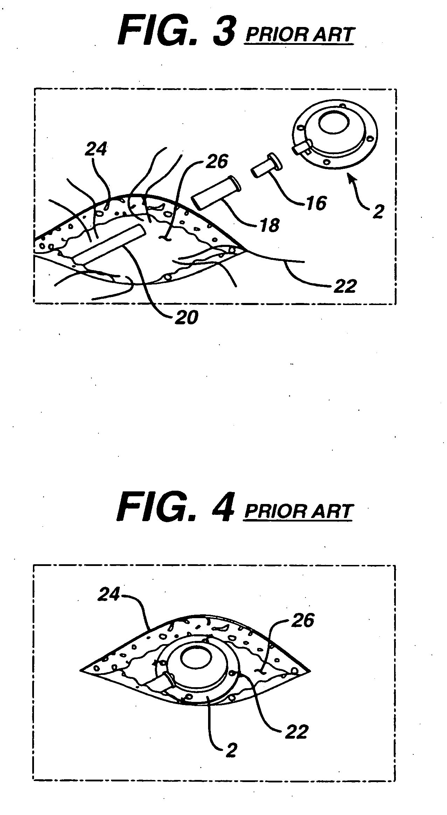

FIG. 3 shows injection port 2 of the prior art as it may be assembled to a catheter 20 during a surgical procedure. When using injection port 2 in a laparoscopic procedure such as implantation of a gastric band, it may be necessary for the surgeon to assemble injection port 2 to catheter 20 during the laparoscopic procedure. This may be because injection port 2 may be too large to pass through a standard size (12mm diameter) laparoscopic port, which may be used for access to the stomach inside the abdominal fluid reservoir. The surgeon may introduce the gastric band and catheter 20 into the abdominal fluid reservoir without injection port 2 attached to the free end of catheter 20. Once the surgeon has secured the gastric band around the stomach, the surgeon externalizes the free end of catheter 20 through the abdominal muscle and fascia layers, subcutaneous fat layer, and the skin to assemble injection port 20 to the free end of catheter 20. Then the surgeon implants the injection port subcutaneously at the desired location on the patient's abdomen. As shown in FIG. 3, a catheter element 16 fits over catheter 20 and locks catheter 20 tightly over catheter support 8 of injection port 2. A catheter protector 18 also fits over catheter 20 and helps to prevent accidental puncture of catheter 20 when the surgeon accesses injection port 2 with a hypodermic needle during later injections of fluid. Once catheter 20 is fluidly connected to injection port 2, the surgeon attaches injection port 2 with a plurality of sutures 22 to the fascia 26 covering the muscular layer of tissue. Typically the surgeon spends several minutes to suture injection port 2 to fascia 26, working with limited access through an incision 24 in the patient. FIG. 4 shows injection port 2 attached to fascia 26 with four sutures 22 prior to closure of incision 24.

The below embodiments describe an injection port that may be configurable into a collapsed or an undeployed position to facilitate placement into the tissue of the patient, and may be configurable, once positioned in the tissue of the patient, into an extended or a deployed position for long-term stability. The injection port resists "flipping" over, thereby allowing needle access to the septum for adding or withdrawing fluid, and provides sites for tissue in-growth for securing the injection port in the tissue of the patient. Furthermore, these embodiments eliminate the need to suture the injection port to tissue, thereby reducing surgery time and the tissue trauma associated with suturing.

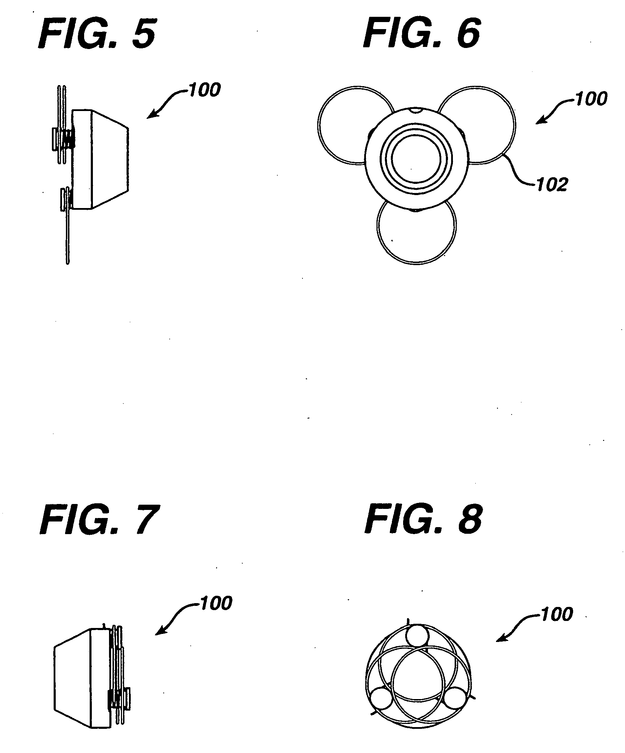

FIGS. 5, 6, 7, 8, and 9 show a first embodiment of an injection port 100, which includes a housing 104 having a body 107 made of a rigid material such as titanium, stainless steel, or a biocompatible polymer. Housing 104 may be of a similar design as housing 14 of the prior art shown in FIG. 1, but without flange 6. A plurality of stability elements 102 attach to housing 104. Each of stability elements 102 include a member 103 that may be made of coiled, metallic wire, preferably a non-corroding, stainless steel or titanium alloy spring wire such as used for the manufacture of coiled springs. Each of stability elements 102 have a torsion spring 105 that attaches member 103 to housing 104 such that stability elements 102 tend to spring from the undeployed position to the deployed position when not sufficiently restrained. FIG. 5 is a side view and FIG. 6 is a top view of injection port 100 while stability elements 102 are in a deployed position. FIG. 7 is a side view and FIG. 8 is a bottom view of injection port 100 while stability elements 102 are in an undeployed position. The surgeon may hold stability elements 102 in the undeployed position with a surgical grasper or gloved hand and then place injection port 100 into the incision of the patient. Once the surgeon has placed injection port 100 in the desired implant location of the patient, the surgeon may release injection port 100 so that stability elements 102 move to the deployed position. The surgeon may use conventional surgical tools to dissect tissue around injection port 100 and facilitate the full extension of stability elements 102.

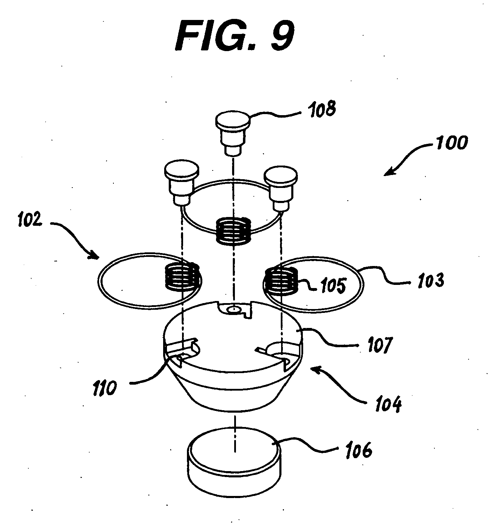

FIG. 9 is an exploded, perspective view of injection port 100. Each of stability elements 102 comprises member 103 and torsion spring 105 that springably attaches to housing 104 with a pin 108 pressed into a hole 110. The space inside of member 103 allows the dissected tissue planes to heal together, thus helping to secure injection port 102 in the patient. Since each of stability elements 102 may be flexible and resiliently attached to housing 104, the patient will not experience significant discomfort while bending/twisting that portion of his or her body. A septum 106 assembles into housing 104 in a similar manner as shown in FIG. 1 of the prior art. (Each of the embodiments of injection port disclosed herein include a septum, a fluid reservoir, and a catheter support having a basic design and function similar to that of the prior art injection port described for Fig. 1.)

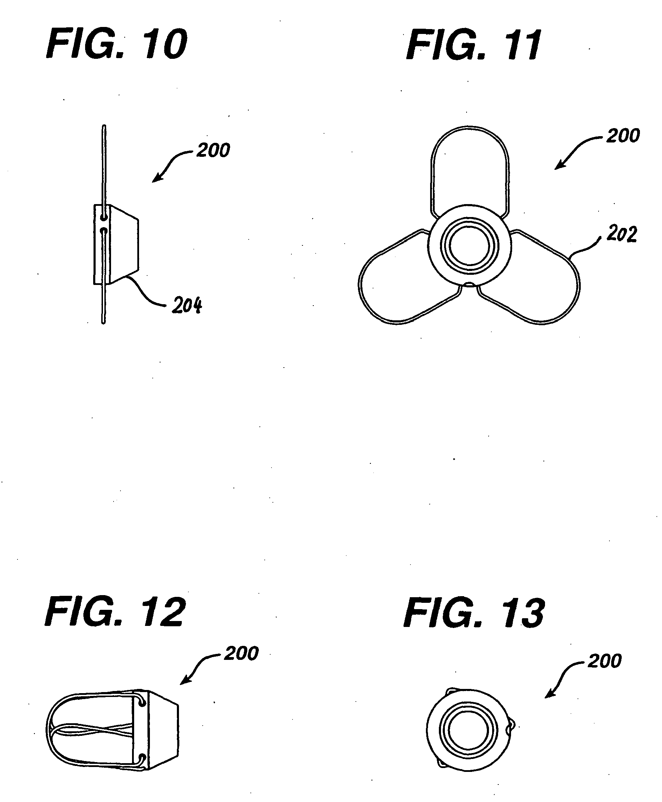

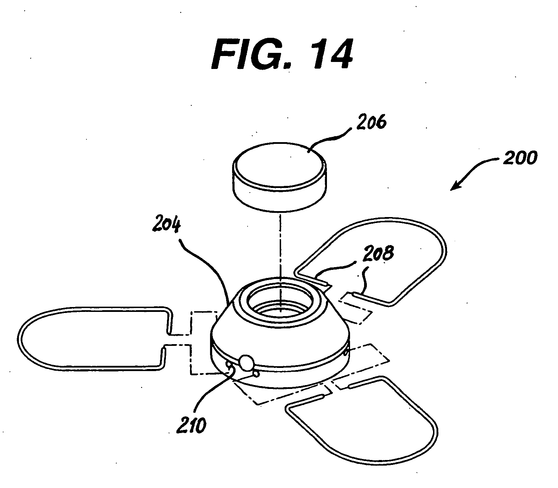

FIGS. 10, 11, 12, 13, and 14 show a second embodiment of an injection port 200. FIG. 10 is a side view, and FIG. 11 is a top view of injection port 200 while in a deployed position. FIG. 12 is a side view, and FIG. 13 is a top view of injection port 200 while in an undeployed position. FIG. 14 is an exploded, perspective view of injection port 200, including a plurality of stability elements 202 made of a metallic wire. Each of stability elements 202 may have a pair of ends 208 that pivotally attach to a housing 204 in holes 210. A septum 206 assembles into housing 204 in a similar manner as shown in FIG. 1 of the prior art. In this embodiment, each of stability elements 202 may be D-shaped. Initially, the surgeon may hold housing 204 with a grasper or gloved hand while injection port 202 may be in the undeployed position. As the surgeon pushes injection port 200 into the tissue of the patient, stability elements 202 unfold into the deployed position while simultaneously penetrating into tissue. Therefore, the surgeon dissects the minimal amount of tissue to position injection port 200, thus facilitating rapid healing and reducing the risk of infection. The subcutaneous fat layer and skin layers cover and hold injection port 200 while tissue heals around stability elements 202.

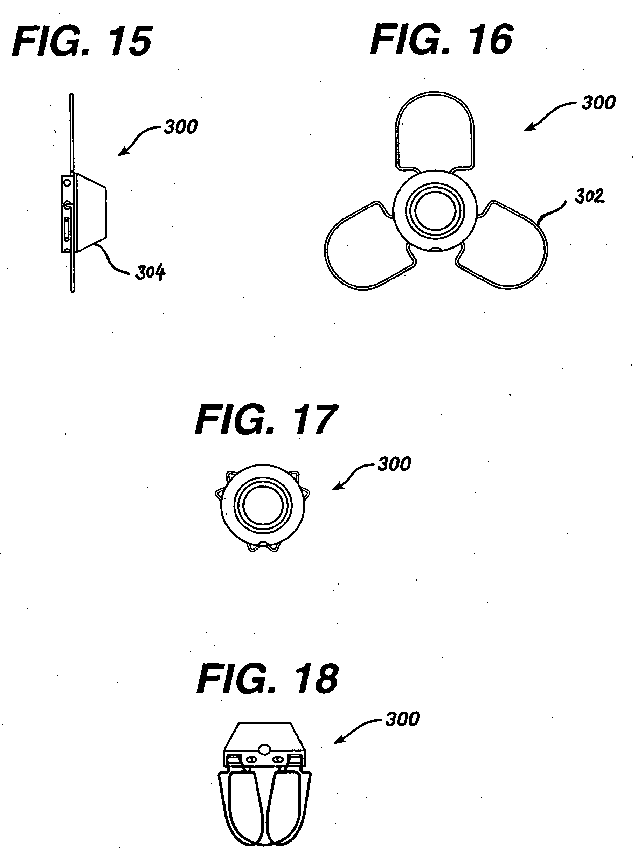

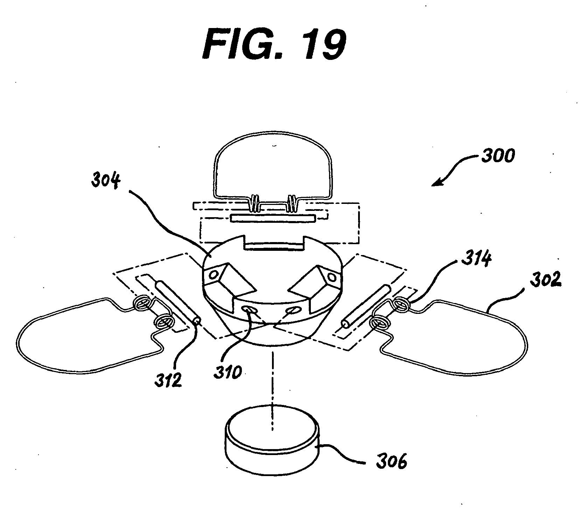

FIGS. 15, 16, 17, 18, and 19 show a third embodiment of an injection port 300. FIG. 15 is a side view, and FIG. 16 is a top view, of injection port 300 while in a deployed position. FIG. 17 is a top view, and FIG. 18 is a side view of injection port 300 while in an undeployed position. FIG. 19 is an exploded, perspective view of injection port 300, including a plurality of stability elements 302 that are made of a spring metal wire. Each of stability elements 302 may have a D-shape as in the previous embodiment, but may be also formed to have torsion springs 314 that attach to a housing 304 with a pin 312 into holes 310 so that stability element 302 may be in the deployed position when unrestrained. The surgeon may place injection port 302 into the tissue of a patient in a similar manner as described for injection port 200 of FIG. 14. A septum 306 assembles into housing 304 as described for the prior art of FIG. 1.

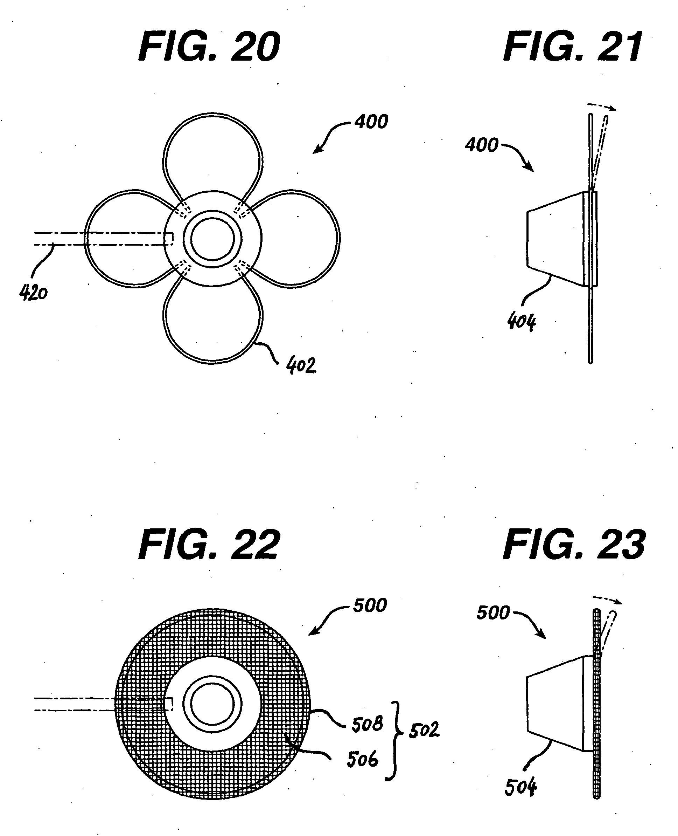

FIG. 20 is a top view and FIG. 21 is a side view of a fourth embodiment of an injection port 400, which includes a plurality of stability elements 402 attached to a housing 404. Stability elements 402 are made of a flexible wire, such as super elastic, nickel-titanium memory metal, also known in the art as Nitinol. The surgeon may hold stability elements in the undeployed position while positioning injection port into the tissue of the patient, and then use a surgical tool or fingertips to gently position stability elements 402 in the deployed position. FIG. 20 also shows a phantom view of a catheter 420 for fluid transfer to a remote portion of the body.

FIG. 22 is a top view and FIG. 23 is a side view of a fifth embodiment of an injection port 500, that includes a stability element 502 attached to a housing 504. Stability element 502 comprises a support member 508 that may be made of a flexible metal wire or plastic cord that may be attached to and forms the perimeter of a circular webbing 506. Webbing 506 may be made of a biocompatible, polymeric mesh material such as *Prolene* (Trademark, Ethicon, Inc.) that attaches to housing 504 with a biocompatible adhesive. Webbing 506 provides a site for rapid tissue in-growth and healing, and to comfortably secure injection port 500 in the body.

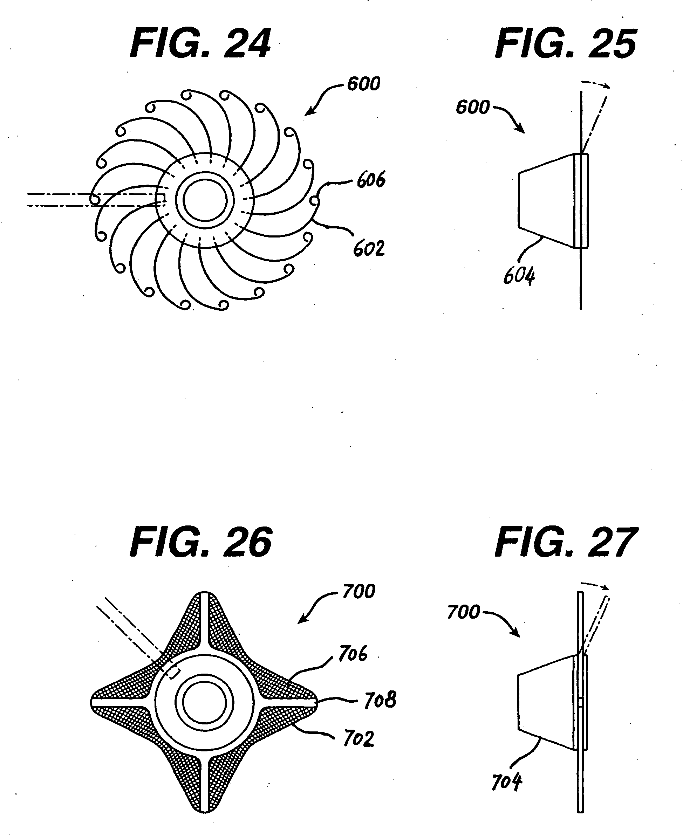

FIG. 24 is a top view and FIG. 25 is a side view of a sixth embodiment of an injection port 600, that includes a plurality of stability elements 602 attached to a housing 604 and normally extending radially. Each of stability elements 602 is made of a flexible metal wire material such as super elastic nickel titanium alloy, and includes a curled end 606.

FIG. 26 is a top view and FIG. 27 is a side view of a seventh embodiment of an injection port 700, that includes a stability element 702 attached to a housing 704. Stability element 702 includes a flexible, star-shaped webbing 706 that may be injection molded from a plastic such as polyethylene with a plurality of support members 708 extending radially. An annular groove 705 of housing 704 retains stability element 702.

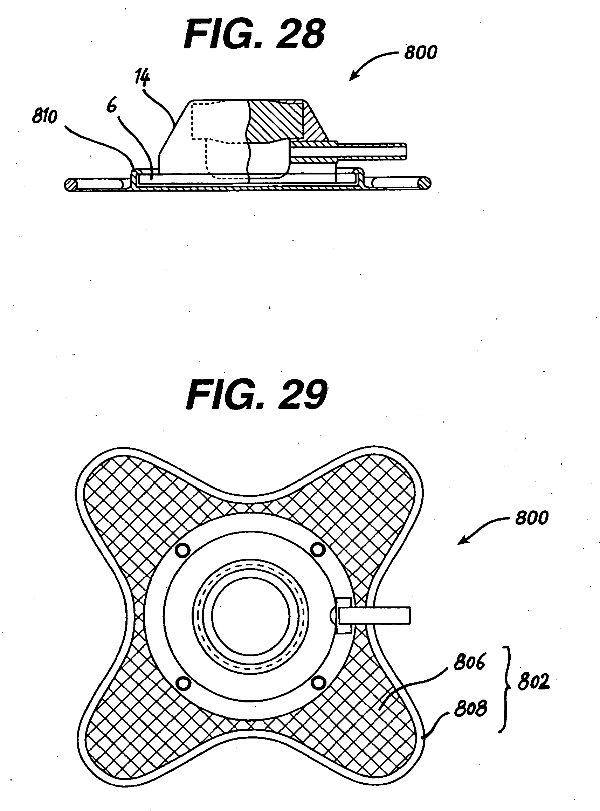

FIG. 28 is a side, sectional view and FIG. 29 is a top view of an eighth embodiment of an injection port 800, that includes a stability element 802. In this embodiment, the surgeon or a medical assistant may assemble injection port 2 of the prior art (FIG. 1) with stability element 802 during the surgical procedure (but prior to placement in the body.) Stability element 802 includes a webbing 806 integrally molded from a flexible, biocompatible plastic such as polyethylene, with a support member 808 that defines the perimeter of stability element 802. A retaining lip 810, also molded into stability element 802, snaps over and retains flange 6 of housing 14. Therefore it may be possible for a surgeon to use a conventional injection port that comes with a particular medical implant device, together with stability element 802, to avoid the need to suture the injection port to tissue.

A surgeon may implant an injection port in accordance with the present invention into the tissue of a surgical patient, without the need for suturing. The surgeon may create a surgical incision through the skin and subcutaneous fat layers of the patient. In the case of a gastric band implant, this incision may be typically made in the abdomen of the patient. The surgeon dissects tissue in the surgical incision to create space for a catheter and the injection port between the subcutaneous fat layer and the fascia tissue. The surgeon may use conventional surgical tools for dissection and/or fingertips. The surgeon connects the injection port to the catheter using components such as described for the prior art in FIG. 1. The surgeon holds the injection port in an undeployed position, and then positions the injection port and the catheter through the incision. The surgeon manipulates the injection port into final position upon the fascia tissue while allowing the injection port to change into a deployed position. Finally, the surgeon or medical assistant closes the skin and subcutaneous fat layers over the injection port and the catheter. The method may also include an additional step of suturing the stabilizing elements to the tissue.

It will become readily apparent to those skilled in the art that the above invention has equally applicability to other types of implantable bands. For example, bands are used for the treatment of fecal incontinence. One such band is described in U.S. Patent 6,461,292 which is hereby incorporated herein by reference. Bands can also be used to treat urinary incontinence. One such band is described in U.S. Patent Application 2003/0105385 which is hereby incorporated herein by reference. Bands can also be used to treat heartburn and/or acid reflux. One such band is described in U.S. Patent 6,470,892 which is hereby incorporated herein by reference. Bands can also be used to treat impotence. One such band is described in U.S. Patent Application 2003/0114729.

While preferred embodiments of the present invention have been shown and described herein, it will be obvious to those skilled in the art that such embodiments are provided by way of example only. Numerous variations, changes, and substitutions will now occur to those skilled in the art without departing from the invention. For example, as would be apparent to those skilled in the art, the disclosures herein have equal application in robotic-assisted surgery. In addition, it should be understood that every structure described above has a function and such structure can be referred to as a means for performing that function. Accordingly, it is intended that the invention be limited only by the scope of the appended claims.

## Claims

1. An implantable surgical injection port (100;200;300;400;500;600;700;800) comprising:
a. a housing (104;204;304;404;504;604;704;14) having a body, a closed distal end, an open proximal end, and a fluid reservoir therebetween;
b. a needle penetrable septum (106;206;306) retained in said open proximal end of said housing; and
c. at least one stabilizing element (102;202;302;402;502;602;702;802) mounted to said housing for stabilizing said port within tissue, said stabilizing element comprising a member having an undeployed position and a deployed position,
**characterized in that** said at least one stabilizing element extends radially from said body and, when deployed, lies in a plane that is either
i. substantially parallel to the septum, or
ii. substantially coplanar with said closed distal end of said housing.

2. The injection port of Claim 1, wherein said stability element is flexible.

3. The injection port of Claim 1, wherein said stabilizing element is resilient and is attached to said housing so that said stabilizing element is in said deployed position when relaxed and not subjected to a restraining force.

4. The injection port of Claim 1, wherein said stabilizing element pivotally attaches to said housing.

5. The injection port of Claim 1, wherein said stabilizing element is at least partially made of a metallic wire.

6. The injection port of Claim 1, wherein said stabilizing element is at least partially made of a biocompatible polymer.

7. The injection port of Claim 1, wherein said stabilizing element comprises a flexible webbing (506) adapted for tissue in-growth, said webbing attached to a flexible support element (508) attached to said housing.

8. The injection port of Claim 1, wherein said stabilizing element is removably attachable to said housing.

9. The injection port of Claim 1, wherein said stabilizing element includes means for penetrating into tissue as the surgeon presses said injection port into position, and as said injection port changes from said undeployed position to said deployed position.

10. The injection port of Claim 1 including at least three stabilizing elements mounted to said housing.

11. The injection port of Claim 1, wherein said at least one stability element, when deployed, lies in a plane that is substantially coplanar with said closed distal end of said housing, further comprising
a means for said at least one stability element to penetrate into tissue as the surgeon presses said injection port into position, and as said injection port changes from said undeployed position to said deployed position;

12. The injection port of Claim 11 including at least three stabilizing elements mounted to said housing.

## Patentansprüche

1. Implantierbare chirurgische Injektionsöffnung (100; 200; 300; 400; 500; 600; 700; 800), die Folgendes umfasst:
a. ein Gehäuse (104; 204; 304; 404; 504; 604; 704; 14) mit einem Korpus, einem geschlossenen distalen Ende, einem offenen proximalen Ende und einem Flüssigkeitsreservoir dazwischen;
b. ein mit einer Nadel penetrierbares Septum (106; 206; 306), das im offenen proximalen Ende des Gehäuses gehalten ist; und
c. zumindest ein Stabilisierungselement (102; 202; 302; 402; 502; 602; 702; 802), das zur Stabilisierung der Öffnung im Gewebe a Gehäuse befestigt ist, wobei das Stabilisierungselement ein Glied mit einer nicht abgelegten Stellung und einer abgelegten Stellung umfasst,
**dadurch gekennzeichnet dass** das zumindest eine Stabilisierungselement sich radial vom Korpus aus erstreckt und nach der Ablage in einer Ebene liegt, die entweder
i. im Wesentlichen parallel zum Septum ist oder
ii. im Wesentlichen koplanar mit dem geschlossenen distalen Ende des Gehäuses ist.

2. Injektionsöffnung nach Anspruch 1, worin das Stabilisierungselement flexibel ist.

3. Injektionsöffnung nach Anspruch 1, worin das Stabilisierungselement nachgiebig und am Gehäuse befestigt ist, so dass das Stabilisierungselement sich in der abgelegten Stellung befindet, wenn es entspannt ist und keinen Haltekräften unterworfen ist.

4. Injektionsöffnung nach Anspruch 1, worin das Stabilisierungselement schwenkbar am Gehäuse befestigt ist.

5. Injektionsöffnung nach Anspruch 1, worin das Stabilisierungselement zumindest teilweise aus einem Metalldraht besteht.

6. Injektionsöffnung nach Anspruch 1, worin das Stabilisierungselement zumindest teilweise aus einem biokompatiblen Polymer besteht.

7. Injektionsöffnung nach Anspruch 1, worin das Stabilisierungselement ein flexibles Gewebe (506) umfasst, in das Gewebe einwachsen kann, wobei das Gewebe an einem am Gehäuse befestigten flexiblen Stützelement (508) befestigt ist.

8. Injektionsöffnung nach Anspruch 1, worin das Stabilisierungselement entfernbar am Gehäuse befestigt ist.

9. Injektionsöffnung nach Anspruch 1, worin das Stabilisierungselement ein Mittel zur Penetration in Gewebe, wenn der Chirurg die Injektionsöffnung in die richtige Stellung drückt und wenn sich die Injektionsöffnung von der nicht abgelegten Stellung in die abgelegte Stellung verändert, umfasst.

10. Injektionsöffnung nach Anspruch 1 mit zumindest drei Stabilisierungselementen, die am Gehäuse befestigt sind.

11. Injektionsöffnung nach Anspruch 1, worin das zumindest eine Stabilisierungselement nach der Ablage in einer Ebene liegt, die im Wesentlichen koplanar mit dem geschlossenen distalen Ende des Gehäuses ist, ferner umfassend ein Mittel für das zumindest eine Stabilisierungselement zur Penetration in Gewebe, wenn der Chirurg die Injektionsöffnung in die richtige Stellung drückt und wenn sich die Injektionsöffnung von der nicht abgelegten Stellung in die abgelegte Stellung verändert.

12. Injektionsöffnung nach Anspruch 11 mit zumindest drei Stabilisierungselementen, die am Gehäuse befestigt sind.

## Revendications

1. Orifice (100 ; 200 ; 300 ; 400 ; 500 ; 600 ; 700 ; 800) d'injection chirurgical implantable comportant :
a. un boîtier (104 ; 204 ; 304 ; 404 ; 504 ; 604 ; 704 ; 14) muni d'un corps, une extrémité distale fermée, une extrémité proximale ouverte, et un réservoir de fluide entre les deux ;
b. un septum (106 ; 206 ; 306) pénétrable par aiguille retenu dans ladite extrémité proximale ouverte dudit boîtier ; et
c. au moins un élément (102 ; 202 ; 302 ; 402 ; 502 ; 602 ; 702 ; 802) monté sur ledit boîtier pour stabiliser ledit orifice dans les tissus, ledit élément de stabilisation comportant un organe ayant une position non déployée et une position déployée,
**caractérisé en ce que** ledit au moins un élément de stabilisation s'étend radialement depuis ledit corps et, lorsque déployé, repose dans un plan qui est soit
i. sensiblement parallèle au septum, ou
ii. sensiblement dans le même plan que ladite extrémité distale fermée dudit boîtier.

2. Orifice d'injection selon la revendication 1, dans lequel ledit élément de stabilisation est souple.

3. Orifice d'injection selon la revendication 1, dans lequel ledit élément de stabilisation est résilient et est fixé audit boîtier de façon à ce que ledit élément de stabilisation soit dans ladite position déployée lorsque relâché et non soumis à une force de restriction.

4. Orifice d'injection selon la revendication 1, dans lequel ledit élément de stabilisation se fixe de façon pivotante audit boîtier.

5. Orifice d'injection selon la revendication 1, dans lequel ledit élément de stabilisation est au moins partiellement réalisé dans un fil métallique.

6. Orifice d'injection selon la revendication 1, dans lequel ledit élément de stabilisation est au moins partiellement réalisé dans un polymère biocompatible.

7. Orifice d'injection selon la revendication 1, dans lequel ledit élément de stabilisation comporte une bande (506) souple adaptée à la croissance interne de tissu, ladite bande étant fixée à un élément (508) de support souple fixé audit boîtier.

8. Orifice d'injection selon la revendication 1, dans lequel ledit élément de stabilisation peut être fixé de manière amovible audit boîtier.

9. Orifice d'injection selon la revendication 1, dans lequel ledit élément de stabilisation comprend un moyen de pénétration dans les tissus au fur et à mesure que le chirurgien appuie sur ledit orifice d'injection pour le mettre en place, et au fur et à mesure que ledit orifice d'injection change de ladite position non déployée à ladite position déployée.

10. Orifice d'injection selon la revendication 1 comprenant au moins trois éléments de stabilisation montés sur ledit boîtier.

11. Orifice d'injection selon la revendication 1, dans lequel ledit au moins un élément de stabilisation, lorsque déployé, repose dans un plan qui est sensiblement le même que celui de ladite extrémité distale fermée dudit boîtier, comportant en outre un moyen, pour ledit au moins un élément de stabilisation, lui permettant de pénétrer dans les tissus au fur et à mesure que le chirurgien appuie sur ledit orifice d'injection pour le mettre en place, et au fur et à mesure que ledit orifice d'injection change de ladite position non déployée à ladite position déployée ;

12. Orifice d'injection selon la revendication 11 comprenant au moins trois éléments de stabilisation montés sur ledit boîtier.
